(19) Europäisches Patentamt
European Patent Office
Office européen des brevets

(11) **EP 3 123 936 A1**

(12) **EUROPEAN PATENT APPLICATION**

(43) Date of publication:
**01.02.2017 Bulletin 2017/05**

(51) Int Cl.:
**A61B 5/021** (2006.01)  **A61B 5/04** (2006.01)
**A61B 5/02** (2006.01)  **A61B 5/00** (2006.01)

(21) Application number: **16181352.2**

(22) Date of filing: **27.07.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.07.2015 JP 2015148096**

(71) Applicant: **Nihon Kohden Corporation**
**Shinjuku-ku**
**Tokyo (JP)**

(72) Inventors:
• **ONO, Yoshinobu**
  **Shinjuku-ku, Tokyo (JP)**
• **TAKAYANAGI, Tsuneo**
  **Shinjuku-ku, Tokyo (JP)**
• **KAIAMI, Takashi**
  **Shinjuku-ku, Tokyo (JP)**

(74) Representative: **Grünecker Patent- und**
**Rechtsanwälte**
**PartG mbB**
**Leopoldstraße 4**
**80802 München (DE)**

(54) **VITAL SIGNS INFORMATION MEASURING APPARATUS AND VITAL SIGNS INFORMATION MEASURING METHOD**

(57)    A vital signs information measuring apparatus includes a calculating section which calculates a baroreflex index, a sympathetic nerve index, a heart rate, an estimated cardiac output, and an alternative index of blood pressure by using at least one of an electrocardiographic signal of a living body, and a pulse wave of the living body and a displaying section that displays changes of the baroreflex index, sympathetic nerve index, heart rate, estimated cardiac output, and alternative index of blood pressure that are calculated by the calculating section.

FIG. 1

EP 3 123 936 A1

**Description**

BACKGROUND

The present invention relates to a vital signs information measuring apparatus and a vital signs information measuring method which can comprehensively evaluate the autonomic nervous function and the heart function.

The autonomic nerves include sympathetic nerves which function mainly in an active state, and parasympathetic nerves which function mainly in a resting state. When a living body is in a tense or active state, the sympathetic nerves are in the sympathetic nerve dominant state, and the blood pressure and the pulse rate are raised. When a living body is in a resting state, the sympathetic nerves are in the parasympathetic nerve dominant state, and the blood pressure and the pulse rate are lowered. In this way, the autonomic nervous function and the heart function are closely correlated with each other.

As an apparatus for detecting whether the autonomic nervous function is normal or not, conventionally, known are an autonomic nervous function diagnostic apparatus and autonomic nervous function measuring apparatus which are disclosed in Japanese Patent Nos 5,480,800 and 5,408,751, respectively. As an apparatus for detecting whether the heart function is normal or not, there is a blood volume measuring apparatus which is disclosed in Japanese Patent No. 5,432,765.

When the apparatus disclosed in Japanese Patent No. 5,480,800 or that disclosed in Japanese Patent No. 5,408,751 is used, it is possible to diagnose whether the autonomic nervous function normally operates or not. When the apparatus disclosed in Japanese Patent No. 5,432,765 is used, it is possible to diagnose whether the heart function normally operates or not.

As described above, however, the autonomic nervous function and the heart function mutually influence each other. Therefore, it is preferable to simultaneously diagnose both whether the autonomic nervous function normally operates or not, and whether the heart function normally operates or not. In the case where the cause of a disease called orthostatic hypotension is to be detected, for example, it is preferable to simultaneously measure both the autonomic nervous function and the heart function.

The orthostatic hypotension disease shows a symptom that, immediately after rising up from the lying state or the sitting state, the blood pressure is largely lowered, and dizziness or syncope occurs. Immediately after rising up from the lying state, usually, the blood pressure in the head is temporarily lowered because the head is located above the position of the heart. Immediately after rising up from the sitting state, the blood pressure in the head is temporarily lowered by influences of the acceleration and gravity acting on the heart and the blood. In a usual case, it is expected that the reduction of the blood pressure is rapidly detected, the activity of the heart is revitalized, and the blood pressure is quickly raised. In a patient with orthostatic hypotension, however, the blood pressure is not quickly raised, and dizziness or syncope occurs.

The cause of the symptom that the blood pressure is hardly raised immediately after the patient rises can be more clarified by simultaneously measuring the autonomic nervous function and the heart function. Conventionally, however, there is no apparatus which can simultaneously measure the autonomic nervous function and the heart function.

The invention has been conducted in order to solve the problem with the prior art. It is an object of the invention to provide a vital signs information measuring apparatus and method which can comprehensively evaluate the autonomic nervous function and the heart function.

SUMMARY

According to an aspect of the invention, a vital signs information measuring apparatus includes a calculating section which calculates a baroreflex index, a sympathetic nerve index, a heart rate, an estimated cardiac output, and an alternative index of blood pressure by using at least one of an electrocardiographic signal of a living body, and a pulse wave of the living body and a displaying section that displays changes of the baroreflex index, sympathetic nerve index, heart rate, estimated cardiac output, and alternative index of blood pressure that are calculated by the calculating section.

BRIEF DESCRIPTION OF DRAWINGS

Fig. 1 is a block diagram of a vital signs information measuring apparatus of an embodiment.
Fig. 2 is a block diagram of a calculator illustrated in Fig. 1.
Fig. 3 is a flowchart illustrating a procedure of a vital signs information measuring method of the embodiment.
Fig. 4 is a subroutine flowchart illustrating a procedure of calculation of a baroreflex index in step S160 of the flowchart illustrated in Fig. 3.

Fig. 5 is a subroutine flowchart illustrating a procedure of calculation of a sympathetic nerve index in step S160 of the flowchart illustrated in Fig. 3.

Fig. 6 is a subroutine flowchart illustrating a procedure of calculation of the heart rate in step S160 of the flowchart illustrated in Fig. 3.

Fig. 7 is a subroutine flowchart illustrating a procedure of calculation of an estimated cardiac output in step S160 of the flowchart illustrated in Fig. 3.

Fig. 8 is a subroutine flowchart illustrating a procedure of calculation of a alternative index of blood pressure in step S160 of the flowchart illustrated in Fig. 3.

Fig. 9 is a view illustrating an electrocardiogram waveform, a pulse wave, the RR interval, and the PWTT.

Fig. 10 is a view illustrating modes of displaying the baroreflex index, sympathetic nerve index, heart rate, estimated cardiac output, and alternative index of blood pressure which are finally obtained by the vital signs information measuring apparatus and method of the embodiment.

## DETAILED DESCRIPTION OF EMBODIMENTS

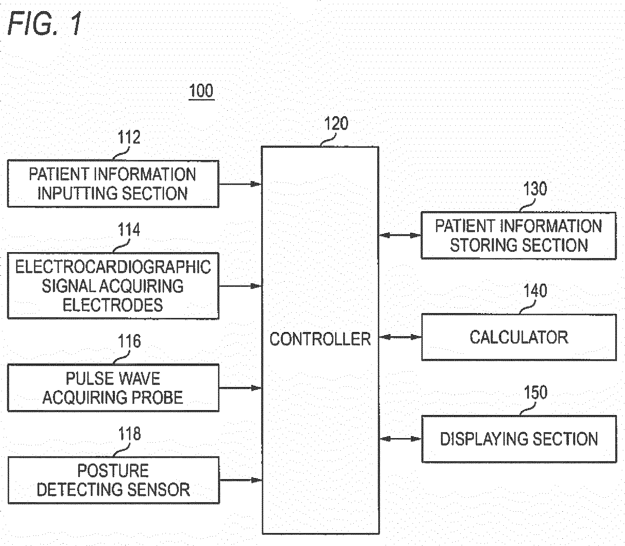

Next, the vital signs information measuring apparatus and method of the invention will be described in detail with reference to the drawings. Fig. 1 is a block diagram of a vital signs information measuring apparatus of an embodiment.

The vital signs information measuring apparatus 100 of the embodiment has a patient information inputting section 112, electrocardiographic signal acquiring electrodes 114, a pulse wave acquiring probe 116, a posture detecting sensor 118, a controller 120, a patient information storing section 130, a calculator 140, and a displaying section 150.

The name, age, and sex of the patient who is to be subjected to the measurement by the vital signs information measuring apparatus 100 are input to the patient information inputting section 112. Also the cardiac output in a resting state of the patient which is measured by using the thoracic impedance method is input to the patient information inputting section 112. The patient information which is input to the patient information inputting section 112 is stored in the patient information storing section 130 through the controller 120.

The patient information inputting section 112 may be an inputting device which is to be operated by an operator, such as a keyboard or a mouse, or an interface to which an external computer is connected.

The electrocardiographic signal acquiring electrodes 114 are attached to the body surface of the patient to acquire the electrocardiographic signal of the patient. Usually, the electrocardiographic signal acquiring electrodes 114 are attached to six portions, namely, the right and left wrists, the right and left ankles, and the right and left chests. The electrocardiographic signal acquired by the electrocardiographic signal acquiring electrodes 114 is supplied to the calculator 140 through the controller 120.

The pulse wave acquiring probe 116 has a clip-like shape, and is attached to the fingertip of the hand of the patient to acquire the pulse wave of the patient. The pulse wave acquired by the pulse wave acquiring probe 116 is supplied to the calculator 140 through the controller 120.

The posture detecting sensor 118 is attached to the body surface of the patient to detect the posture of the patient by using an acceleration change. Postures which can be detected by the posture detecting sensor 118 are static states such as the supine position, the sitting position, and the standing position, and dynamic states such as a change from the supine position or the sitting position to the standing position, and that from the standing position to the supine position or the sitting position.

The controller 120 controls individually and comprehensively the operations of the patient information inputting section 112, electrocardiographic signal acquiring electrodes 114, pulse wave acquiring probe 116, posture detecting sensor 118, patient information storing section 130, calculator 140, and displaying section 150 which constitute the vital signs information measuring apparatus 100.

The patient information storing section 130 stores the patient information which is supplied from the patient information inputting section 112. For example, the patient information includes the name, age, and sex of the patient, and the cardiac output in a resting state of the patient. Since the patient information storing section 130 stores the cardiac output in a resting state of the patient, the calculator 140 can calculate an estimated cardiac output.

The calculator 140 calculates a baroreflex index, a sympathetic nerve index, the heart rate, the estimated cardiac output, and an alternative index of blood pressure, by using the electrocardiographic signal of the patient which is acquired by the electrocardiographic signal acquiring electrodes 114, the pulse wave of the patient which is acquired by the pulse wave acquiring probe 116, the posture of the patient which is detected by the posture detecting sensor 118, and the cardiac output in a resting state of the patient which is supplied from the patient information inputting section 112.

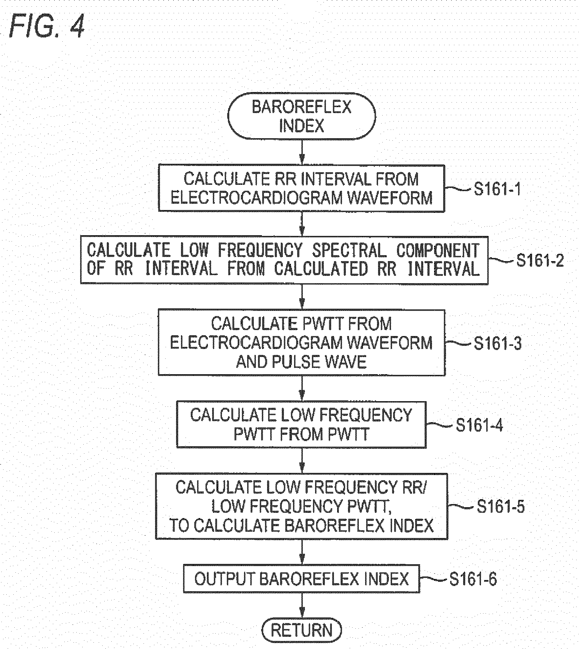

The baroreflex index is an index which indicates the sensitivity of a function of holding the blood pressure in a fixed range, and which relates to the autonomic nervous function. The sympathetic nerve index is an index which relates to an increase of the heart rate, and which relates to the autonomic nervous function. The heart rate is a number at which the heart beats for a fixed period of time, and an index which relates to the heart function. The estimated cardiac output is an estimated amount of blood which is carried out from the heart, and an index which relates to the heart function. The alternative index of blood pressure is the so-called PWTT (Pulse Wave Transit Time), and an index which relates to the heart function. Figs. 4 to 8 illustrate procedures of calculating the baroreflex index, the sympathetic nerve index, the heart rate, the estimated cardiac output, and the alternative index of blood pressure, respectively. The procedures will be described later in detail.

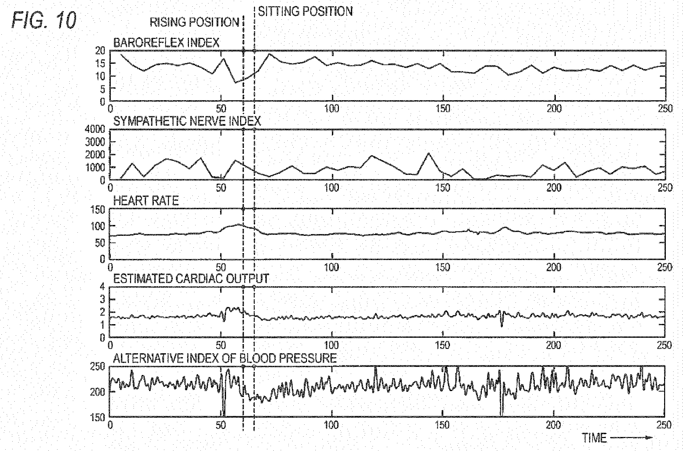

The displaying section 150 displays graphically and time sequentially changes of the baroreflex index, sympathetic nerve index, heart rate, estimated cardiac output, and alternative index of blood pressure which are calculated by the calculator 140. When the five indexes are displayed graphically and time sequentially, the doctor can comprehensively evaluate in an easy manner the autonomic nervous function and the heart function. Fig. 10 illustrates manners of displaying the baroreflex index, the sympathetic nerve index, the heart rate, the estimated cardiac output, and the alternative index of blood pressure. The display manners will be described later in detail.

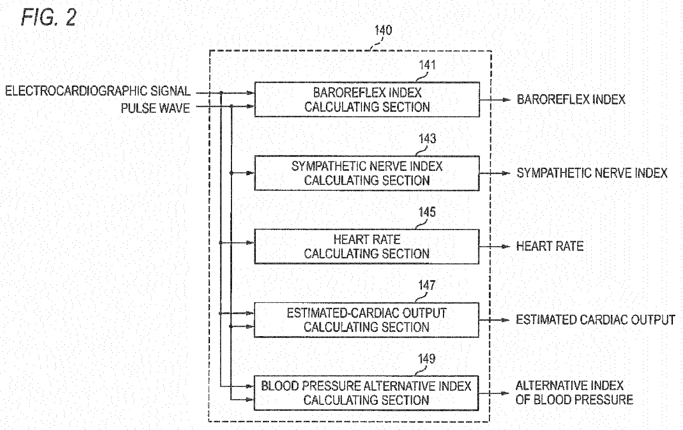

Fig. 2 is is a block diagram of the calculator 140 illustrated in Fig. 1.

The calculator 140 may include a baroreflex index calculating section 141, a sympathetic nerve index calculating section 143, a heart rate calculating section 145, an estimated-cardiac output calculating section 147, and a blood pressure alternative index calculating section 149.

The baroreflex index calculating section 141 calculates the baroreflex index by using the electrocardiographic signal acquired by the electrocardiographic signal acquiring electrodes 114, and the pulse wave acquired by the pulse wave acquiring probe 116. Specifically, the baroreflex index calculating section 141 calculates the low frequency spectral component of RR interval of the electrocardiogram waveform by using the electrocardiographic signal, and the low frequency PWTT by using the electrocardiogram waveform and the pulse wave. Furthermore, the baroreflex index is calculated by calculating a ratio of the low frequency spectral component of RR interval to the low frequency PWTT (hereinafter, the ratio is referred to as Low frequency spectral component of RR interval/Low frequency PWTT).

When the baroreflex index calculating section 141 calculates the baroreflex index, it is possible to determine the degree of the sensitivity of a function of holding the blood pressure in a fixed range.

The sympathetic nerve index calculating section 143 calculates the sympathetic nerve index by using the electrocardiographic signal acquired by the electrocardiographic signal acquiring electrodes 114. Specifically, the sympathetic nerve index calculating section 143 calculates the low frequency spectral component of RR interval and high frequency spectral component of RR interval of the electrocardiogram waveform by using the electrocardiographic signal, and further calculates a ratio of the low frequency spectral component of RR interval to the high frequency spectral component of RR interval (hereinafter, the ratio is referred to as Low frequency RR/High frequency RR), thereby calculating the sympathetic nerve index.

When the sympathetic nerve index calculating section 143 calculates the sympathetic nerve index, it is possible to determine the degree of the autonomic nervous function relating to the increase of the heart rate.

The heart rate calculating section 145 calculates the heart rate by using the electrocardiographic signal. The heart rate is a number at which the heart beats for a fixed period of time, and therefore it is possible to determine the degree of the heart function.

The estimated-cardiac output calculating section 147 calculates the heart rate by using the electrocardiographic signal which is acquired in a resting state of the living body by the electrocardiographic signal acquiring electrodes 114, and further calculates the PWTT by using the electrocardiographic signal and pulse wave in a resting state of the living body. Moreover, the estimated-cardiac output calculating section 147 calculates an estimated-cardiac output calculation coefficient by using the cardiac output in a resting state of the living body which is stored in the patient information storing section 130, and the calculated heart rate and PWTT. Then, the estimated-cardiac output calculating section 147 calculates the heart rate by using the electrocardiographic signal in a load state of the living body, calculates the PWTT by using the electrocardiographic signal and pulse wave in a load state of the living body, and calculates an estimated cardiac output by using the heart rate in a load state of the living body, the PWTT, and the above-described estimated-cardiac output calculation coefficient.

Therefore, the estimated-cardiac output calculating section 147 can calculate the estimated cardiac output which is an estimated amount of blood that is carried out from the heart in a load state of the patient, and can determine the degree of the heart function.

The blood pressure alternative index calculating section 149 calculates an alternative index of blood pressure by using the electrocardiographic signal acquired by the electrocardiographic signal acquiring electrodes 114, and the pulse wave acquired by the pulse wave acquiring probe 116. Specifically, the blood pressure alternative index calculating section 149 calculates the PWTT by using the electrocardiographic signal and the pulse wave, and outputs the PWTT as the alternative index of blood pressure. The alternative index of blood pressure relates to the heart function, and therefore it is possible to determine the degree of the heart function.

As described above, the calculator 140 can obtain the five indexes, i.e., the baroreflex index, the sympathetic nerve index, the heart rate, the estimated cardiac output, and the alternative index of blood pressure. Therefore, the doctor can comprehensively evaluate the autonomic nervous function and the heart function.

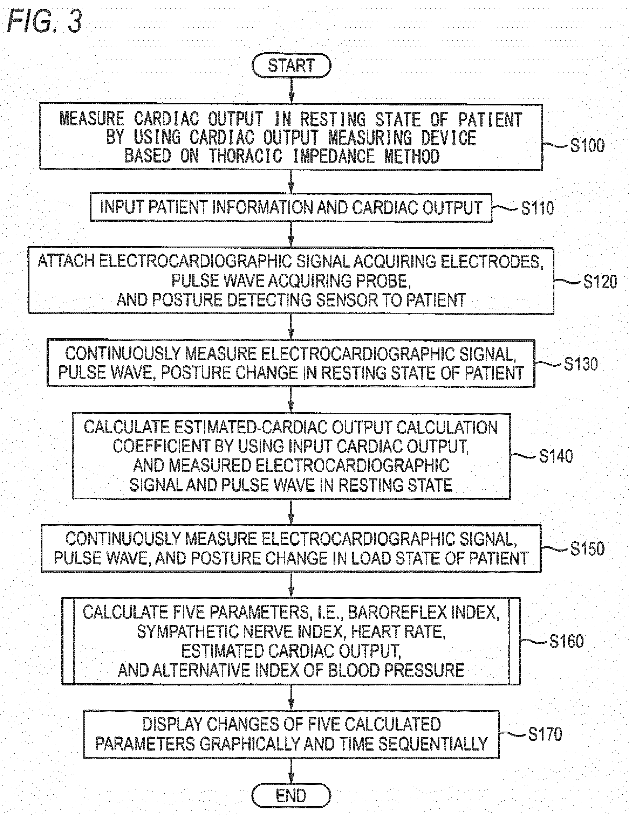

Fig. 3 is a flowchart illustrating the procedure of the vital signs information measuring method of the invention. The flowchart is also a flowchart illustrating the operation of the vital signs information measuring apparatus 100 of the invention.

First, the cardiac output in a resting state of the patient is measured by using a cardiac output measuring device based on the thoracic impedance method (step S100). Various methods are known as a method of measuring the cardiac output in a resting state of the patient. In the embodiment, a non-invasive continuous method of measuring the cardiac output based on the thoracic impedance method can be performed. The cardiac output in a resting state of the patient is measured by using, for example, a task force monitor.

Next, the patient information and the cardiac output are input (step S110). Specifically, the name, age, and sex of the patient, and the cardiac output in a resting state of the patient which is measured in step S100 are input through the patient information inputting section 112.

The electrocardiographic signal acquiring electrodes 114, the pulse wave acquiring probe 116, and the posture detecting sensor 118 are attached to the patient (step S120). Specifically, total six electrocardiographic signal acquiring electrodes 114 are attached to the right and left wrists, right and left ankles, and right and left chests of the patient, respectively, one pulse wave acquiring probe 116 is attached to the fingertip of the hand of the patient, and one posture detecting sensor 118 is attached to the lumbar part of the patient.

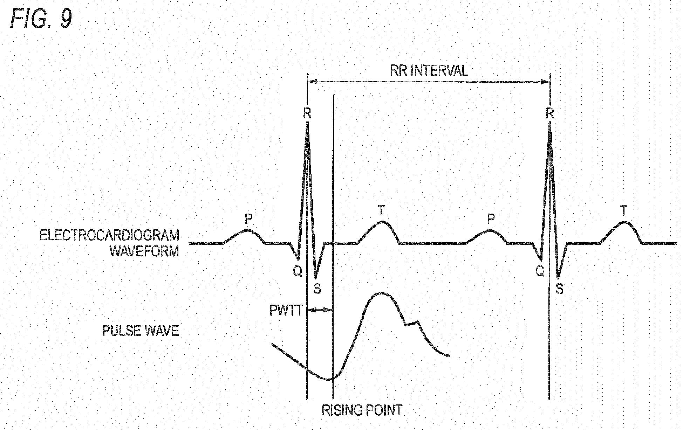

Next, the electrocardiographic signal, pulse wave, posture change in a resting state of the patient are continuously measured (step S130). In a state where the patient lies, specifically, a change of the electrocardiographic signal is continuously measured, that of the pulse wave is continuously measured, and that of the posture is continuously measured. As a result of the measurements, as seen from the graphs illustrated in Fig. 9 which is a view illustrating the electrocardiogram waveform, the pulse wave, the RR interval, and the PWTT, for example, it is possible to obtain time sequential changes of the electrocardiogram waveform and the pulse wave. In a resting state, the posture is not largely changed.

The estimated-cardiac output calculation coefficient is calculated by using the input cardiac output, and the measured electrocardiographic signal and pulse wave in a resting state (step S140). Specifically, the PWTT and the heart rate are calculated by using the cardiac output in a resting state of the patient which is input in step S110 from the patient information inputting section 112, and the electrocardiographic signal and pulse wave in a resting state which are measured in step S130, and the estimated-cardiac output calculation coefficient is calculated by using the PWTT and heart rate which are calculated.

The cardiac output is the amount of blood which is carried out from the heart, and an index for measuring the heart function. When the cardiac output is indicated by Co, the PWTT is indicated by PW, and the heart rate is indicated by HR, the cardiac output Co is expressed by the following expression.

output Co is expressed by the following expression.

$$Co = K \cdot (\alpha \cdot PW + \beta) \cdot HR.$$

When Co indicating the cardiac output, PW indicating the PWTT, and HR indicating the heart rate are known, therefore, the estimated-cardiac output calculation coefficients K, $\alpha$, and $\beta$ can be calculated by using the least squares method.

Next, the electrocardiographic signal, pulse wave, posture change in a load state of the patient are continuously measured (step S150). The load state means a change of the posture of the patient from the supine position or the sitting position to the standing position, or from the standing position to the supine position or the sitting position.

In this step, therefore, changes of the electrocardiographic signal and the pulse wave are continuously measured when the posture of the patient is changed from the supine position or the sitting position to the standing position. On the contrary, also changes of the electrocardiographic signal and the pulse wave can be continuously measured when the posture of the patient is changed from the standing position to the supine position or the sitting position.

The calculator 140 calculates five parameters, i.e., the baroreflex index, sympathetic nerve index, heart rate, estimated cardiac output, and alternative index of blood pressure in an arbitrary posture of the patient by using at least one of the electrocardiographic signal and pulse wave in a load state of the patient which are measured in step S150 (step S160). The procedures of calculating the five parameters will be described later in detail with reference to the flowcharts of Figs. 4 to 8.

The displaying section 150 displays changes of the five parameters, i.e., the baroreflex index, sympathetic nerve index, heart rate, estimated cardiac output, and alternative index of blood pressure which are calculated by the calculator 140, graphically and time sequentially as shown in, for example, Fig. 10 (step S170).

Fig. 4 is a subroutine flowchart illustrating the procedure of calculating the baroreflex index in step S160 of the flowchart illustrated in Fig. 3.

The baroreflex index calculating section 141 draws an electrocardiogram waveform such as illustrated in Fig. 9 by using the electrocardiographic signal in a load state of the patient, and calculates the interval between the R waves of two heart beats of the electrocardiogram waveform, i.e., the RR interval (step S161-1). Preferably, the RR interval is obtained with respect to all of heart beats which are continuously measured.

Usually, the RR interval which is calculated in step S161-1 is not constant among all heart beat intervals, and fluctuates with the heart beat intervals. Therefore, the baroreflex index calculating section 141 calculates the low frequency spectral component of RR interval which is a low-frequency component of the fluctuation, from the calculated RR interval (step S161-2). In the calculation of the low frequency spectral component of RR interval, a conventionally commonly used frequency analysis method such as the FFT is employed.

Next, the baroreflex index calculating section 141 draws an electrocardiogram waveform and pulse wave such as illustrated in Fig. 9 by using the electrocardiographic signal and pulse wave in a load state of the patient, and calculates the time difference between the peak of the R wave of the electrocardiogram waveform and the rising of the pulse wave, i.e., the PWTT (step S161-3).

Usually, the PWTT which is calculated in step S161-3 is not constant in all heart beats, and fluctuates with heart beats. Therefore, the baroreflex index calculating section 141 calculates the low frequency PWTT which is a low-frequency component of the fluctuation, from the calculated PWTT (step S161-4). In the calculation of the low frequency PWTT, a conventionally commonly used frequency analysis method such as the FFT is employed.

The baroreflex index calculating section 141 calculates Low frequency RR/Low frequency PWTT by using the low frequency spectral component of RR interval which is calculated in step S161-2, and the low frequency PWTT which is calculated in step S161-4, to calculate the baroreflex index (step S161-5).

The baroreflex index calculating section 141 outputs the calculated baroreflex index to the displaying section 150 (step S161-6). The displaying section 150 displays graphically and time sequentially a change of the baroreflex index.

When the baroreflex index is calculated, it is possible to determine the degree of the sensitivity of the function of holding the blood pressure in a fixed range.

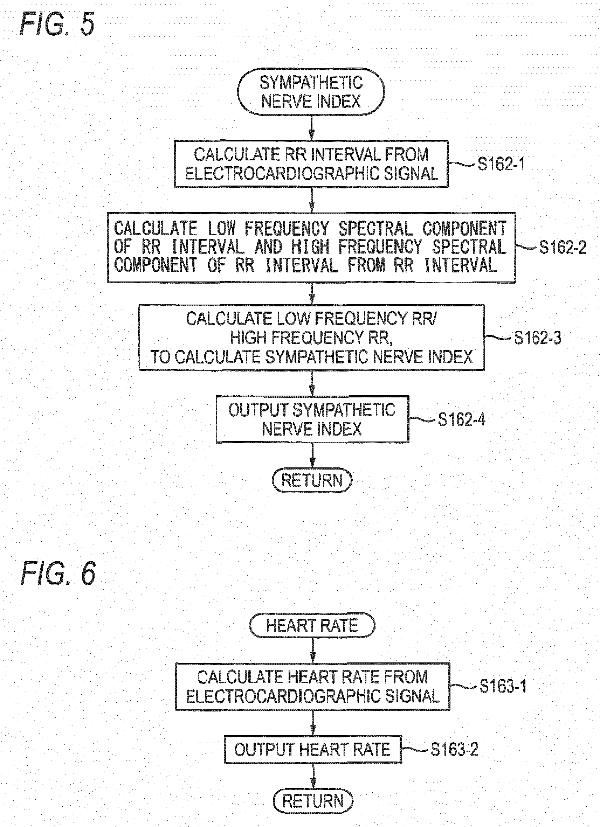

Fig. 5 is a subroutine flowchart showing the procedure of calculating the sympathetic nerve index in step S160 of the flowchart illustrated in Fig. 3.

The sympathetic nerve index calculating section 143 calculates the RR interval from the electrocardiographic signal in a load state of the patient in a procedure similar to that of above-described step S161-1 (step S162-1).

Same or similarly with above-described step S161-2, the sympathetic nerve index calculating section 143 calculates the low frequency spectral component of RR interval which is a low-frequency component of the fluctuation of the calculated RR interval, from the calculated RR interval, and further calculates the high frequency spectral component of RR interval which is a high-frequency component of the fluctuation of the RR interval (step S162-2). In the calculation of the high frequency spectral component of RR interval, a conventionally commonly used frequency analysis method such as the FFT is employed.

The sympathetic nerve index calculating section 143 calculates Low frequency RR/High frequency RR by using the low frequency spectral component of RR interval and high frequency spectral component of RR interval which are calculated in step S162-2 to calculate the sympathetic nerve index (step S162-3).

The sympathetic nerve index calculating section 143 outputs the calculated sympathetic nerve index to the displaying section 150 (step S162-4). The displaying section 150 displays graphically and time sequentially a change of the sympathetic nerve index.

When the sympathetic nerve index is calculated, it is possible to determine the degree of the balance of the autonomic nervous function.

Fig. 6 is a subroutine flowchart showing the procedure of calculation of the heart rate in step S160 of the flowchart illustrated in Fig. 3.

The heart rate calculating section 145 draws an electrocardiogram waveform by using the electrocardiographic signal in a load state of the patient, and calculates the heart rate from the electrocardiogram waveform. The heart rate is calculated by checking the number at which the electrocardiogram waveform (P-Q-R-S-T wave) such as shown in Fig. 9 occurs for a fixed period of time (step S163-1).

The heart rate calculating section 145 outputs the calculated heart rate to the displaying section 150 (step S163-2). The displaying section 150 displays graphically and time sequentially a change of the heart rate.

When the heart rate is calculated, it is possible to determine the degree of the heart function.

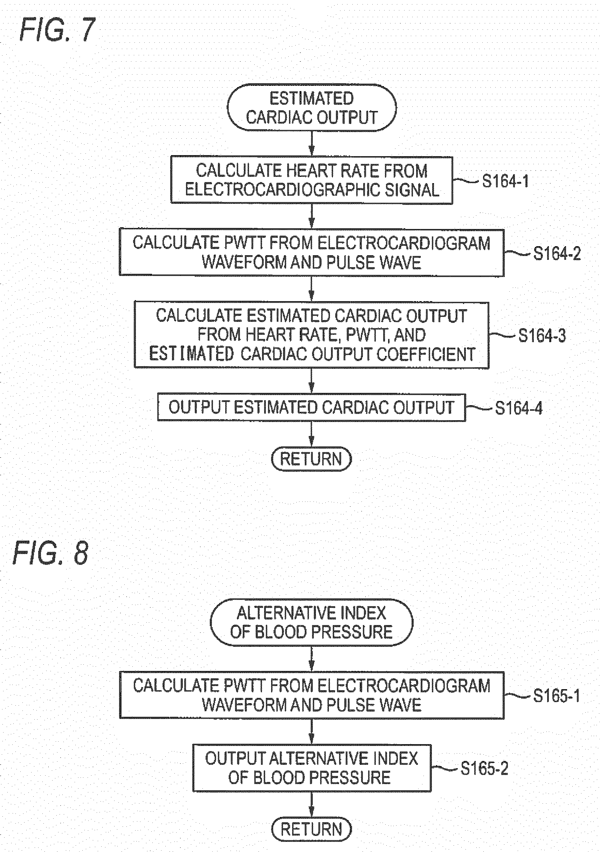

Fig. 7 is a subroutine flowchart illustrating the procedure of calculating the estimated cardiac output in step S160 of the flowchart illustrated in Fig. 3.

Similarly with step S163-1, the estimated-cardiac output calculating section 147 draws an electrocardiogram waveform by using the electrocardiographic signal in a load state of the patient, and calculates the heart rate from the electrocardiogram waveform (step S164-1).

Similarly with step S161-3, the estimated-cardiac output calculating section 147 draws an electrocardiogram waveform and pulse wave such as illustrated in Fig. 9 by using the electrocardiographic signal and pulse wave in a load state of the patient, and calculates the PWTT from the time difference between the peak of the R wave of the electrocardiogram waveform and the rising of the pulse wave (step S164-2).

The estimated-cardiac output calculating section 147 calculates the estimated cardiac output in a load state of the patient from the heart rate which is calculated in step S164-1, the PWTT which is calculated in step S164-2, and the estimated cardiac output coefficient which is calculated in step S140 (step S164-3).

When the cardiac output is indicated by Co, the PWTT is indicated by PW, and the heart rate is indicated by HR, as described above, the estimated cardiac output Co' is expressed by Co' = K x ($\alpha$ x PW + P) x HR. The estimated-cardiac output calculation coefficients K, $\alpha$, and $\beta$ are calculated in step S140 by using the least squares method. When the heart rate HR which is calculated in step S164-1, and the PWTT which is calculated in step S164-2 are substituted into the above expression, therefore, the estimated cardiac output Co' can be calculated.

The estimated-cardiac output calculating section 147 outputs the calculated estimated cardiac output to the displaying section 150 (step S164-4). The displaying section 150 displays graphically and time sequentially a change of the estimated cardiac output.

The estimated cardiac output is an estimated amount of blood which is carried out from the heart in a load state of the patient, and an index which relates to the heart function. When the estimated cardiac output is calculated, therefore, it is possible to determine the degree of the heart function.

Fig. 8 is a subroutine flowchart illustrating the procedure of calculating the alternative index of blood pressure in step S160 of the flowchart illustrated in Fig. 3.

Similarly with step S161-3, the blood pressure alternative index calculating section 149 draws an electrocardiogram waveform and pulse wave such as illustrated in Fig. 9 by using the electrocardiographic signal and pulse wave in a load state of the patient, and calculates the PWTT from the time difference between the peak of the R wave of the electrocardiogram waveform and the rising of the pulse wave (step S165-1).

The blood pressure alternative index calculating section 149 outputs the calculated PWTT to the displaying section 150 (step S165-2). The displaying section 150 displays graphically and time sequentially a change of the PWTT.

The PWTT is an index which relates to the heart function. When the PWTT is calculated, therefore, it is possible to determine the degree of the heart function.

Fig. 10 illustrates modes of displaying the baroreflex index, sympathetic nerve index, heart rate, estimated cardiac output, and alternative index of blood pressure which are finally obtained by the vital signs information measuring apparatus and method of the embodiment.

The display of Fig. 10 is performed by the displaying section 150. Fig. 10 sequentially illustrates from the top changes of: the baroreflex index calculated by the baroreflex index calculating section 141; the sympathetic nerve index calculated by the sympathetic nerve index calculating section 143; the heart rate calculated by the heart rate calculating section 145; the estimated cardiac output calculated by the estimated-cardiac output calculating section 147; and the alternative index of blood pressure calculated by the blood pressure alternative index calculating section 149. The graphs are displayed while their abscissas or time axes coincide with one another.

In Fig. 10, the posture of the patient is changed in the temporal sequence of the sitting position (seating position) → the rising position → the standing position → the sitting position. The rising in Fig. 10 is a timing when the patient rises from the sitting position to the standing position, and the sitting is that when the patient sits down from the standing position to the sitting position.

In Fig. 10, a starting point on which attention is to be focused is the rising position, and an ending point on which attention is to be focused is the time zone in which the level returns to a level identical with that in a resting state. When changes of the heart rate in these timings are considered, the heart rate in the rising position is increased, and, after the seating position, the level returns to a level identical with that in a resting state which is before the rising position. From these changes, it is seen that the patient shows a normal biological response.

It is known that a healthy person who is rising shows the following biological response. When the posture is first changed from the sitting position to the standing position, i.e., when the person rises, temporary hypotension occurs. As a result, the baroreflex works, and the baroreflex index is increased. Next, sympathetic nerves work, the sympathetic nerve index is increased, and the heart rate is raised. As a result, the cardiac output is increased, and the estimated cardiac output is increased. Then, the blood pressure is raised, and the alternative index of blood pressure is lowered.

A healthy person who is rising shows the above-described biological response. When the five indexes are simultaneously displayed side by side as in Fig. 10, therefore, the autonomic nervous function and the heart function can be comprehensively evaluated in an easy manner.

Although the vital signs information measuring apparatus and method of the invention have been described in one embodiment, it is a matter of course that the technical concept of the vital signs information measuring apparatus and method of the invention is not limited to the embodiment.

**Claims**

1. A vital signs information measuring apparatus comprising:

   a calculating section which calculates a baroreflex index, a sympathetic nerve index, a heart rate, an estimated cardiac output, and an alternative index of blood pressure by using at least one of an electrocardiographic signal of a living body, and a pulse wave of the living body; and
   a displaying section that displays changes of the baroreflex index, sympathetic nerve index, heart rate, estimated cardiac output, and the alternative index that are calculated by the calculating section.

2. The vital signs information measuring apparatus according to claim 1, wherein the calculating section includes:

   a baroreflex index calculating section that calculates a baroreflex index by using the electrocardiographic signal and the pulse wave;
   a sympathetic nerve index calculating section that calculates a sympathetic nerve index by using the electrocardiographic signal;
   a heart rate calculating section that calculates a heart rate by using the electrocardiographic signal;
   an estimated-cardiac output calculating section that calculates an estimated cardiac output by using the electrocardiographic signal and the pulse wave; and
   a blood pressure alternative index calculating section that calculates an alternative index of blood pressure by using the electrocardiographic signal and the pulse wave.

3. The vital signs information measuring apparatus according to claim 2 further comprising a storage that stores therein a cardiac output in a resting state of the living body.

4. The vital signs information measuring apparatus according to claim 2 or 3, wherein the baroreflex index calculating section calculates a low frequency spectral component of RR interval of an electrocardiogram waveform by using the electrocardiographic signal, and a low frequency PWTT by using the electrocardiogram waveform and the pulse wave, and further calculates Low frequency RR/Low frequency PWTT, thereby calculating the baroreflex index.

5. The vital signs information measuring apparatus according to claim 2 or 3, wherein the sympathetic nerve index calculating section calculates the low frequency spectral component of RR interval and high frequency spectral component of RR interval of an electrocardiogram waveform by using the electrocardiographic signal, and further calculates Low frequency RR/High frequency RR, thereby calculating the sympathetic nerve index.

6. The vital signs information measuring apparatus according to claim 3, wherein the estimated-cardiac output calculating section calculates:

   the heart rate by using the electrocardiographic signal in a resting state of the living body;
   a PWTT by using the electrocardiographic signal and pulse wave in a resting state of the living body; and
   an estimated-cardiac output calculation coefficient by using the cardiac output in a resting state of the living body which is stored in the storing section, and the calculated heart rate and PWTT, and

   the estimated-cardiac output calculating section further calculates:

   the heart rate by using an electrocardiographic signal in a load state of the living body;
   the PWTT by using the electrocardiographic signal and pulse wave in a load state of the living body; and
   an estimated cardiac output by using the heart rate in a load state of the living body, the PWTT, and the estimated-cardiac output calculation coefficient.

7. The vital signs information measuring apparatus according to claim 2 or 3, wherein the blood pressure alternative index calculating section calculates a PWTT by using the electrocardiographic signal and the pulse wave, and outputs the PWTT as the alternative index of blood pressure.

8. The vital signs information measuring apparatus according to any one of claims 1 to 7, wherein the displaying section displays changes of the baroreflex index, the sympathetic nerve index, the heart rate, the estimated cardiac output, and the alternative index of blood pressure, graphically and time sequentially.

9. A vital signs information measuring method comprising:

   calculating an estimated-cardiac output calculation coefficient by using a cardiac output, electrocardiographic signal, and pulse wave in a resting state of a living body;
   calculating a baroreflex index, a sympathetic nerve index, a heart rate, and an alternative index of blood pressure by using at least one of an electrocardiographic signal and pulse wave in a load state of the living body, and calculating an estimated cardiac output of the living body by using the electrocardiographic signal and pulse wave in a load state of the living body, and the estimated-cardiac output calculation coefficient; and
   displaying changes of the baroreflex index, sympathetic nerve index, heart rate, estimated cardiac output, and alternative index of blood pressure which are calculated.

10. The vital signs information measuring method according to claim 9, wherein the baroreflex index is obtained by calculating a low frequency spectral component of RR interval of an electrocardiogram waveform by using the electrocardiographic signal, and a low frequency PWTT by using the electrocardiogram waveform and the pulse wave, and further calculating Low frequency RR/Low frequency PWTT.

11. The vital signs information measuring method according to claim 9, wherein the sympathetic nerve index is obtained by calculating a low frequency spectral component of RR interval and high frequency spectral component of RR interval of an electrocardiogram waveform by using the electrocardiographic signal, and further calculating Low frequency RR/High frequency RR.

12. The vital signs information measuring method according to claim 9, wherein the estimated cardiac output is obtained by calculating the heart rate by using the electrocardiographic signal, calculating a PWTT by using the electrocardiographic signal and the pulse wave, and using the heart rate, the PWTT, and the estimated-cardiac output calculation coefficient.

13. The vital signs information measuring method according to claim 9, wherein the alternative index of blood pressure is obtained by calculating a PWTT by using the electrocardiographic signal and the pulse wave.

*FIG. 1*

100

112
PATIENT INFORMATION
INPUTTING SECTION

114
ELECTROCARDIOGRAPHIC
SIGNAL ACQUIRING
ELECTRODES

116
PULSE WAVE
ACQUIRING PROBE

118
POSTURE
DETECTING SENSOR

120
CONTROLLER

130
PATIENT INFORMATION
STORING SECTION

140
CALCULATOR

150
DISPLAYING SECTION

FIG. 2

140

ELECTROCARDIOGRAPHIC SIGNAL
PULSE WAVE

141
| BAROREFLEX INDEX CALCULATING SECTION | → BAROREFLEX INDEX

143
| SYMPATHETIC NERVE INDEX CALCULATING SECTION | → SYMPATHETIC NERVE INDEX

145
| HEART RATE CALCULATING SECTION | → HEART RATE

147
| ESTIMATED-CARDIAC OUTPUT CALCULATING SECTION | → ESTIMATED CARDIAC OUTPUT

149
| BLOOD PRESSURE ALTERNATIVE INDEX CALCULATING SECTION | → ALTERNATIVE INDEX OF BLOOD PRESSURE

## FIG. 3

START

MEASURE CARDIAC OUTPUT IN RESTING STATE OF PATIENT
BY USING CARDIAC OUTPUT MEASURING DEVICE
BASED ON THORACIC IMPEDANCE METHOD — S100

INPUT PATIENT INFORMATION AND CARDIAC OUTPUT — S110

ATTACH ELECTROCARDIOGRAPHIC SIGNAL ACQUIRING ELECTRODES,
PULSE WAVE ACQUIRING PROBE,
AND POSTURE DETECTING SENSOR TO PATIENT — S120

CONTINUOUSLY MEASURE ELECTROCARDIOGRAPHIC SIGNAL,
PULSE WAVE, POSTURE CHANGE IN RESTING STATE OF PATIENT — S130

CALCULATE ESTIMATED-CARDIAC OUTPUT CALCULATION
COEFFICIENT BY USING INPUT CARDIAC OUTPUT,
AND MEASURED ELECTROCARDIOGRAPHIC
SIGNAL AND PULSE WAVE IN RESTING STATE — S140

CONTINUOUSLY MEASURE ELECTROCARDIOGRAPHIC SIGNAL,
PULSE WAVE, AND POSTURE CHANGE IN LOAD STATE OF PATIENT — S150

CALCULATE FIVE PARAMETERS, I.E., BAROREFLEX INDEX,
SYMPATHETIC NERVE INDEX, HEART RATE,
ESTIMATED CARDIAC OUTPUT,
AND ALTERNATIVE INDEX OF BLOOD PRESSURE — S160

DISPLAY CHANGES OF FIVE CALCULATED
PARAMETERS GRAPHICALLY AND TIME SEQUENTIALLY — S170

END

*FIG. 4*

```
                    ┌──────────────────┐
                    │    BAROREFLEX     │
                    │      INDEX        │
                    └────────┬─────────┘
                             │
          ┌──────────────────▼──────────────────┐
          │  CALCULATE RR INTERVAL FROM          │──── S161-1
          │  ELECTROCARDIOGRAM WAVEFORM          │
          └──────────────────┬──────────────────┘
                             │
      ┌──────────────────────▼──────────────────────┐
      │ CALCULATE LOW FREQUENCY SPECTRAL COMPONENT   │──── S161-2
      │ OF RR INTERVAL FROM CALCULATED RR INTERVAL   │
      └──────────────────────┬──────────────────────┘
                             │
          ┌──────────────────▼──────────────────┐
          │      CALCULATE PWTT FROM             │──── S161-3
          │  ELECTROCARDIOGRAM WAVEFORM          │
          │       AND PULSE WAVE                 │
          └──────────────────┬──────────────────┘
                             │
            ┌────────────────▼────────────────┐
            │    CALCULATE LOW FREQUENCY       │──── S161-4
            │      PWTT FROM PWTT              │
            └────────────────┬────────────────┘
                             │
         ┌───────────────────▼───────────────────┐
         │    CALCULATE LOW FREQUENCY RR/         │──── S161-5
         │      LOW FREQUENCY PWTT,               │
         │  TO CALCULATE BAROREFLEX INDEX         │
         └───────────────────┬───────────────────┘
                             │
              ┌──────────────▼──────────────┐
              │ OUTPUT BAROREFLEX INDEX      │──── S161-6
              └──────────────┬──────────────┘
                             │
                       ┌─────▼─────┐
                       │  RETURN   │
                       └───────────┘
```

*FIG. 5*

```
         ┌──────────────────┐
         │   SYMPATHETIC    │
         │   NERVE INDEX    │
         └──────────────────┘
                  │
                  ▼
    ┌─────────────────────────────┐
    │  CALCULATE RR INTERVAL FROM │ ─── S162-1
    │  ELECTROCARDIOGRAPHIC SIGNAL│
    └─────────────────────────────┘
                  │
                  ▼
  ┌──────────────────────────────────────┐
  │ CALCULATE LOW FREQUENCY SPECTRAL      │
  │ COMPONENT OF RR INTERVAL AND HIGH     │ ─── S162-2
  │ FREQUENCY SPECTRAL COMPONENT OF       │
  │ RR INTERVAL FROM RR INTERVAL          │
  └──────────────────────────────────────┘
                  │
                  ▼
  ┌──────────────────────────────────────┐
  │   CALCULATE LOW FREQUENCY RR/         │
  │   HIGH FREQUENCY RR,                  │ ─── S162-3
  │ TO CALCULATE SYMPATHETIC NERVE INDEX  │
  └──────────────────────────────────────┘
                  │
                  ▼
       ┌───────────────────┐
       │ OUTPUT SYMPATHETIC│ ─── S162-4
       │   NERVE INDEX     │
       └───────────────────┘
                  │
                  ▼
            ┌──────────┐
            │  RETURN  │
            └──────────┘
```

*FIG. 6*

```
            ┌──────────────┐
            │  HEART RATE  │
            └──────────────┘
                   │
                   ▼
    ┌──────────────────────────────┐
    │  CALCULATE HEART RATE FROM   │ ─── S163-1
    │  ELECTROCARDIOGRAPHIC SIGNAL │
    └──────────────────────────────┘
                   │
                   ▼
        ┌──────────────────┐
        │ OUTPUT HEART RATE│ ─── S163-2
        └──────────────────┘
                   │
                   ▼
             ┌──────────┐
             │  RETURN  │
             └──────────┘
```

FIG. 7

```
          ┌──────────────────┐
          │    ESTIMATED     │
          │  CARDIAC OUTPUT  │
          └──────────────────┘
                   │
                   ▼
   ┌──────────────────────────────────┐
   │   CALCULATE HEART RATE FROM      │── S164-1
   │  ELECTROCARDIOGRAPHIC SIGNAL     │
   └──────────────────────────────────┘
                   │
                   ▼
   ┌──────────────────────────────────┐
   │ CALCULATE PWTT FROM ELECTROCARDIOGRAM │── S164-2
   │     WAVEFORM AND PULSE WAVE      │
   └──────────────────────────────────┘
                   │
                   ▼
   ┌──────────────────────────────────┐
   │  CALCULATE ESTIMATED CARDIAC OUTPUT │
   │     FROM HEART RATE, PWTT, AND   │── S164-3
   │ ESTIMATED CARDIAC OUTPUT COEFFICIENT │
   └──────────────────────────────────┘
                   │
                   ▼
   ┌──────────────────────────────────┐
   │ OUTPUT ESTIMATED CARDIAC OUTPUT  │── S164-4
   └──────────────────────────────────┘
                   │
                   ▼
              ( RETURN )
```

FIG. 8

```
          ┌──────────────────┐
          │ ALTERNATIVE INDEX │
          │  OF BLOOD PRESSURE │
          └──────────────────┘
                   │
                   ▼
   ┌──────────────────────────────────┐
   │ CALCULATE PWTT FROM ELECTROCARDIOGRAM │── S165-1
   │     WAVEFORM AND PULSE WAVE      │
   └──────────────────────────────────┘
                   │
                   ▼
   ┌──────────────────────────────────┐
   │   OUTPUT ALTERNATIVE INDEX       │── S165-2
   │     OF BLOOD PRESSURE            │
   └──────────────────────────────────┘
                   │
                   ▼
              ( RETURN )
```

FIG. 9

FIG. 10

Europäisches
Patentamt

European
Patent Office

Office européen
des brevets

# EUROPEAN SEARCH REPORT

Application Number

EP 16 18 1352

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/124914 A1 (KUO TERRY B J [TW] ET AL) 14 May 2009 (2009-05-14) * abstract; figure 1 * * the whole document * | 1-13 | INV. A61B5/021 A61B5/04 A61B5/02 A61B5/00 |
| X | US 2003/000873 A1 (KAWAGUCHI KEIZOH [JP]) 2 January 2003 (2003-01-02) * abstract; figure 3 * * paragraphs [0049] - [0054] * * the whole document * | 1-13 | |
| A,D | JP 5 408751 B2 (UNIV TOHOKU; FUKUDA DENSHI KK) 5 February 2014 (2014-02-05) * the whole document * | 1-13 | |
| A | US 2005/222514 A1 (SUGO YOSHIHIRO [JP] ET AL) 6 October 2005 (2005-10-06) * abstract * | 1-13 | |

TECHNICAL FIELDS
SEARCHED     (IPC)

A61B

The present search report has been drawn up for all claims

| Place of search | Date of completion of the search | Examiner |
|---|---|---|
| The Hague | 6 December 2016 | Furlan, Stéphane |

EPO FORM 1503 03.82 (P04C01)

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.............................................................................................

& : member of the same patent family, corresponding
document

# EP 3 123 936 A1

## ANNEX TO THE EUROPEAN SEARCH REPORT
## ON EUROPEAN PATENT APPLICATION NO.

EP 16 18 1352

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2016

| Patent document cited in search report | | | Publication date | Patent family member(s) | | | Publication date |
|---|---|---|---|---|---|---|---|
| US 2009124914 | A1 | | 14-05-2009 | NONE | | | |
| US 2003000873 | A1 | | 02-01-2003 | EP<br>JP<br>US | 1273315<br>2003010318<br>2003000873 | A1<br>A<br>A1 | 08-01-2003<br>14-01-2003<br>02-01-2003 |
| JP 5408751 | B2 | | 05-02-2014 | JP<br>JP | 5408751<br>2013202123 | B2<br>A | 05-02-2014<br>07-10-2013 |
| US 2005222514 | A1 | | 06-10-2005 | US<br>US | 2005222514<br>2007167852 | A1<br>A1 | 06-10-2005<br>19-07-2007 |

EPO FORM P0459

For more details about this annex : see Official Journal of the European Patent Office, No. 12/82

19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5480800 B
- JP 5408751 B
- JP 5432765 B